# EUROPEAN PATENT APPLICATION

(11) **EP 2 471 386 A1**
(43) Date of publication of application: **04.07.2012**
(21) Application number: 10197297.4
(22) Date of filing: 29.12.2010
(51) Int. Cl.: A23L 1/30, A61K 31/23, A61K 9/00, A61K 9/16, A61K 9/20, A61K 9/48

(54) **Cetyl myristate and/or cetyl palmitate suspension formulations**

(71) Applicant: Deva Holding Anonim Sirketi, Kucukcekmece/Istanbul (TR)
(72) Inventor: Haas, Philipp Daniel, 34303, ISTANBUL (TR); Firat, Omer Faruk, 34303, ISTANBUL (TR); Kandemir, Levent, 34303, ISTANBUL (TR); Koc, Fikret, 34303, ISTANBUL (TR); Sivasligil, Ramazan, 34303, ISTANBUL (TR)

(57) **Abstract**

This invention is related to bilayer tablet, inlay tablet, tablet in tablet, tablet in capsule, capsule in capsule, separated granulation and kit of combination of cetyl myristate and cetyl palmitate.

## Description

### Technical Field

This invention is related to bilayer tablet, inlay tablet, tablet in tablet, tablet in capsule, capsule in capsule, separated granulation and kit of combination of cetyl myristate and cetyl palmitate.

### Background Art

Cetyl palmitate is derived from the fatty acid, palmitic acid which occurs as the glycerol ester in many oils and fats such as palm oil or Chinese vegetable tallow. A synthetic method of preparation is to react palmitoyl chloride and cetyl alcohol in the presence of magnesium. See the Merck Index, 12th edition at page 336. Reference is also made to **US** US3169099 A (SOCONY MOBIL OIL CO INC) 02.09.1965 patent which discloses a biosynthetic method of producing cetyl palmitate.

US 4,113,881 A (DIEHL HARRY WELDON) 12.09.1978 discloses that the administration of an effective amount of cetyl myristoleate to a mammal is useful in inhibiting or relieving the symptoms of inflammatory rheumatoid arthritis in mammals.

US 5569676 A (DIEHL, HARRY W) 29.10.1996 US 5,569,676 discloses the use of cetyl myristoleate in the treatment of osteo-arthritis.

WO 01/85162 A (MERACOL CORP LTD ET.AL.) 15.11.2001 discloses the use of cetyl myristate and/or cetyl palmitate in the treatment of irritable bowel syndrome or disease. Patent embraces that cetyl myristate comprises 50-98 wt.% of the mixture, preferably, the myristate and palmitate are in a weight ratio of 95:5. The oral dosage unit is a capsule and contains 5-400 mg of the cetyl myristate or the mixture of the cetyl myristate and the cetyl palmitate. It also includes an excipient and/or diluent, preferably silicon dioxide, calcium phosphate and/or magnesium oxide. Preferably said mixture is in a capsule and it includes a pharmaceutically acceptable excipient and/or diluents. Preferably the dosage unit includes silicon dioxide, calcium phosphate and/or magnesium oxide. Liquid formulation, where an amount of liquid equivalent to at least 4 capsules is prescribed which is to be taken 3 times daily. That is 4200 mg of cetyl myristate or the mixture of cetyl myristate and cetyl palmitate.That mixture comprises by weight 95% cetyl myristate and 5% cetyl palmitate by weight In addition added excipients were present in the non gelatin two part capsule case.

WO 01/85163 A (MERACOL CORP LTD ET.AL.) 15.11.2001 discloses the use of cetyl myristate and/or cetyl palmitate in the treatment of eczema and/or psoriasis. Accordingly, capsule also includes a pharmaceutically acceptable excipient and/or diluent. These are silicon dioxide, calcium phosphate and/or magnesium oxide. The dosage unit can also be a wax-like solid or can be an orally consumable liquid composition (eg; made up with a general pharmacy type carrier such as methyl cellulose).

WO 2005/118070 A (MERACOL CORP LTD ET.AL.) 15.12.2005 discloses the treatment of multiple sclerosis with the use of cetyl myristate and/or cetyl palmitate.The cetyl myristate; or combination of cetyl myristate and cetyl palmitate is administered simultaneously, separately or sequentially.

WO 03/018731 A (MERACOL CORP LTD) 06.03.2003 defines the process preparing a mixture of cetyl myristate (50-98 wt.%) and cetyl palmitate, for use in the formulation of cosmetics and pharmaceuticals.

WO 03/045374 A (MERACOL CORP LTD ET.AL.) 05.06.2003 discloses the use of cetyl myristate and/or cetyl palmitate in a method of treatment and/or prophylaxis of a mammal for at least the symptoms of treating asthma, chronic obstructive pulmonary disease and/or other respiratory difficulties.

WO 03/026640 A (MERACOL CORP LTD ET.AL.) 03.04.2003 discloses the use of cetyl myristate and/or cetyl palmitate in the treatment of food allergies and/or food intolerances.

WO 01/85164 A (MERACOL CORP LTD ET.AL.) 15.11.2001 discloses the use of cetyl myristate and/or cetyl palmitate in the treatment of herpes.

### Summary of invention

This invention embraces separated formulations and separated granulations of cetyl myristate and cetyl palmitate combination. Accordingly it provides bilayer tablet, inlay tablet, tablet in tablet, capsule in capsule, separated granulation and kit of cetyl myristate and cetyl palmitate.

### Technical Problem

Combination of cetyl myristate and cetyl palmitate can be used as active pharmaceutical ingredients (API) in pharmaceutical and/or dietary supplement formulations. However, generally cetyl palmitate is approximately used 20 times less than cetyl myristate. Disproportionate doses cause dose (content) uniformity problems when they are granulated or treated together. When non separated formulations are created, poor blend uniformity is observed due to agglomeration and/or segregation. Uniformity of low dose solid dosage forms is also impacted granulation methods.

### Solution to Problem

It is invented that uniformity of doses are provided through separated formulations of cetyl myristate and cetyl palmitate. Accordingly, bilayer tablet or inlayed tablet or tablet in tablet or separated granulation or a kit is prepared. Thus effect of cetyl myristate and cetyl palmitate and excipients, on each other, is elected and dose uniformity is achieved.

### Description of embodiments

Homogeneity of the mixture form which is used for manufacturing pharmaceutical or dietary supplement materials is corner stone of the treatment. In other words, proper dose uniformity provides proper treatment.

In accordance with this invention cetyl palmitate is used about 20 times less than cetyl myristate in pharmaceutical and/or dietary supplement formulations, for example 333.3 mg cetyl myristate and 16.7 mg cetyl palmitate. Hence,dose uniformity problem is occured. Structures and material properties of cetyl myristate and cetyl palmitate also impact dose uniformity. Therefore, accompanying granulation of cetyl myristate and cetyl palmitate together mostly gives rise dose non-uniformity.

It is invented that if cetyl myristate and cetyl palmitate are each dispersed within its own pharmaceutically acceptable carrier and then combined in different compartments or separated sections in the pharmaceutical composition or kit.

In one aspect, this invention delineates tablet in tablet formulations. Tablet in tablet comprises an inner tablet that is covered by an outer coat that is compressed onto the inner tablet. Tablet in tablet is sometimes also known as a "compression coated tablet" therefore denomination may be changed.

In one embodiment, the present invention provides tablet-in-tablet compositions comprising: a) a core tablet comprising: cetyl myristate and an acceptable carrier or carriers and b) a compressed outer tablet layer comprising: cetyl palmitate and an acceptable carrier or carriers. Accordingly, core tablet of cetyl myristate is surrounded by outer tablet of cetyl palmitate.

Another embodiment of the present invention is in the form of a tablet-in-tablet wherein the core tablet comprises cetyl palmitate and an acceptable carrier or carriers and the surrounding outer tablet applied thereover comprises cetyl myristate and an acceptable carrier or carriers.

Tablet in tablet formulations can be prepared by techniques known by pharmaceutical industry and can be produced by using of conventional press-coating technology, such as Ima Kilian Tablet Press Model S250P.

Another embodiment of the present invention is to provide inlay tablet. Inlay tablets according to the present invention are tablets wherein inner tablet is positioned within a comparatively larger "outer" tablet in such a way that at least one surface of the inner tablet is not in contact with outer tablet.

Inlay tablet dosage form comprising: (a) an inner inlayed tablet comprising cetyl palmitate and optionally acceptable excipient (s); and (b) an outer tablet comprising cetyl myristate and optionally acceptable excipient(s).

In yet another embodiment inlay tablet dosage form comprising: (a) an inner inlayed tablet comprising cetyl myristate optionally acceptable excipient(s); and (b) an outer tablet comprising cetyl palmitate and optionally acceptable excipient(s).

In another embodiment, this invention embraces bilayer tablet. Bilayer tablet comprises; a) a first layer containing cetyl myristate and optionally acceptable carrier or carriers and b) a second layer containing cetyl palmitate and optionally acceptable carrier or carriers.

In another embodiment, this invention embraces bilayer tablet. Bilayer tablet comprises; a) a first layer containing cetyl palmitate and optionally acceptable carrier or carriers and b) a second layer containing cetyl myristate and optionally acceptable carrier or carriers.

The first and the second layers of bilayer tablet can be arranged side-by-side.

Bilayer tablets can be produced by Korsch XM 12 Bi-Layer Tablet Press.

In another aspect, this invention is directed to a kit. The kit according to the invention comprises multiple separate dosage forms, such as for example, separate capsules or tablets.

In accordance with this invention the kit comprising (i) therapeutically effective amount cetyl palmitate and optionally an acceptable carrier or diluent in a unit dosage form and (ii) therapeutically effective amount of cetyl myristate and an acceptable carrier or diluent in a unit dosage form and (iii) a container.

According to this invention, each unit dosage form is discrete dosage forms that are packaged together. The kits of the invention can include container as separate compositions such as a divided bottle or a divided packet. Separate unit dosage forms can also be contained in undivided container. The pharmaceutical kits can include instructions as a separate sheet or optionally printed on the containers.

Dosage forms in the kit can be simultaneously or sequentially administered.

In still another aspect, this invention embraces capsule in capsule formulations wherein smaller size capsule is encapsulated into a larger capsule. Capsule-in-capsule consists of an external capsule and internal capsule (inner capsule) located therein. It is preferred that smaller size capsule is filled with cetyl palmitate and optionally excipients and larger capsule is filled with cetyl myristate and optionally excipients. It is also preferred that both cetyl myristate and cetyl palmitate are seperately granulated. Capsule in capsule operations can be performed via Modu-C (HarroHofliger).

In another aspect, this invention delineates that cetyl myristate and cetyl palmitate are each dispersed within its own carrier and then combined. In terms of this solution one of the following preparations, for example, can be carried out:
- Cetyl myristate and cetyl palmitate are separately granulated and then they are separately compressed into mini tablets and filled into capsule altogether or
- Cetyl myristate and cetyl palmitate are separately granulated and then they are separately compressed into tablet and filled into capsule or
- One of the active agents, cetyl palmitate or cetyl palmitate, is granulated and compressed into tablet or mini-tablets and then combined with granules of other active agent, cetyl palmitate or cetyl myristate, into capsule or
- Cetyl myristate and cetyl palmitate are separately granulated and then directly filled into capsule without tabletting or
- One of the active agents, cetyl palmitate or cetyl palmitate, is prepared through direct compression into tablet or mini-tablets and then combined with granules of other active agent, cetyl palmitate or cetyl myristate, into capsule.

In another aspect, this invention defines that one of the active agents, cetyl myristate or cetyl palmitate is granulated and then combined with other one, cetyl myristate or cetyl palmitate, which is not in granulated form.

In accordance with present invention, the pharmaceutical or dietary supplement composition comprises cetyl palmitate and cetyl myristate combination, disintegrant, lubricant, binder, filler and the like.

Disintegrants are, but not limited to, alginic acid, chitosan, pectins, cation exchange resins, magnesium silicates, aluminium silicates, mixtures thereof and the like.

Lubricants/Glidants are, but not limited to, magnesium stearate, calcium stearate, hydrogenated castor oil, glyceryl behenate, glyceryl monostearate, glyceryl palmitostearate, leucine, mineral oil, light mineral oil, myristic acid, palmitic acid, polyethylene glycol, potassium benzoate, sodium benzoate, sodium lauryl sulfate, sodium stearyl fumarate, stearic acid, talc, hydrogenated vegetable oil, zinc stearate, magnesium lauryl sulphate, sodium stearyl fumarate, polyethylene glycol, stearic acid, colloidal silicon dioxide or mixtures thereof and the like.

Binders are, but not limited to, sodium alginate, cellulose, methylcellulose, ethylcellulose, hydroxypropyl cellulose, hydroxypropyl methylcellulose, sodium carboxymethyl cellulose, polypropylpyrrolidone, polyvinylprrolidone, gelatin, polyethylene glycol, starch, pre-gelatinized starch, sugars, trehalose, glucose, tragacanth, sorbitol, acacia, alginates, carrageenan, xanthan gum, locust bean gum and gum arabic, waxes, polyacrylamide, mixtures thereof, and the like.

Diluents/fillers are, but not limited to, mannitol, sorbitol, xylitol, microcrystalline cellulose, silicified microcrystalline cellulose, hydroxypropyl methylcellulose, hydroxypropyl cellulose, pullulan and fast dissolving carbohydrates such as Pharmaburst™, mixtures thereof and the like.

### Example 1

**INLAY TABLET**

**A. Inner Tablet**
1. Cetyl palmitate, polyvinylprrolidone, sodium starch glycollate, lactose monohydrate are sieved and sieved material is granulated with a solvent.
2. Granules are dried.
3. Dried granules are sieved.
4. Step 3 materials are blended with magnesium stearate.
5. Lubricated blend is compressed into tablets.

**B. Outer Tablet Blend.**
1. Cetyl myristate, lactose monohydrate, anhydrous lactose, microcrystalline cellulose, colloidal silicon dioxide and croscarmellose sodium are sifted and
2. Povidone is dissolved in ethyl alcohol to prepare a binder solution
3. Sifted material is granulated with binder solution.
4. Granules are dried
5. Dried granules are sieved.
6. Blend is lubricated with magnesium stearate.

**C. Inlay Tablet.**
• Inner tablet is compressed together with the outer tablet blend into inlay tablets.

**Table 4**

| **INLAY TABLET UNIT FORMULA** | |
|---|---|
| **Ingredients** | **mg** |
| **INNER TABLET** | |
| Cetyl Palmitate | 16.7 |
| Polyvinylprrolidone | 7 |
| Sodium starch glycollate | 2 |
| Lactose monohydrate | 12 |
| Magnesium Stearate | 1.2 |

| **OUTER TABLET** | |
|---|---|
| Cetyl Myristate | 333.3 |
| Lactose Monohydrate | 18 |
| Anhydrous lactose | 9 |
| Microcrystalline cellulose | 3.5 |
| Colloidal silicon dioxide | 4.7 |
| Croscarmellose sodium | 1 |
| Povidone | 12 |
| Magnesium Stearate | 3 |
| **Total** | 423.4 |

### Example 2

**BILAYER TABLET**

**A. Layer 1**
1. Cetyl palmitate and polyvinylprrolidone, sodium starch glycollate, lactose monohydrate are sieved and sieved material is granulated with a solvent.
2. Granules are dried.
3. Dried granules are sieved.
4. Blend step 3 materials with magnesium stearate.
5. Lubricated blend is compressed into tablets.

**B. Layer 2**
1. Cetyl myristate, lactose monohydrate, anhydrous lactose, microcrystalline cellulose, colloidal silicon dioxide and croscarmellose sodium are sifted and
2. Povidone is dissolved in ethyl alcohol to prepare a binder solution
3. Sifted material is granulated with binder solution.
4. Granules are dried
5. Dried granules are sieved.
6. Blend is lubricated with magnesium stearate.

**C. Bilayer Tablet.**

Compress Layer 1 blend and Layer 2 together to form bilayer tablets.

**Table 3**

| **BILAYER TABLET UNIT FORMULA** | |
|---|---|
| **Ingredients** | **mg** |
| **LAYER 1** | |
| Cetyl Palmitate | 16.7 |
| Polyvinylprrolidone | 5 |
| Sodium starch glycollate | 3 |
| Lactose monohydrate | 7 |
| Magnesium Stearate | 1 |

| **LAYER 2** | |
|---|---|
| Cetyl Myristate | 333.3 |
| Lactose Monohydrate | 21 |
| Anhydrous lactose | 11 |
| Microcrystalline cellulose | 2.7 |
| Colloidal silicon dioxide | 5 |
| Croscarmellose sodium | 0.5 |
| Povidone | 10 |
| Magnesium Stearate | 2 |
| **Total** | 418.2 |

### Example 3

**TABLET IN TABLET**

**A. Internal Tablet**
1. Cetyl palmitate is sieved.
2. Sieved cetyl palmitate is mixed with Lactose Monohydrate Spray Dried, AVICEL ® PH 101 and HPMC K100M Premium CR in Collette high shear mixer.
3. The blend of step 2 was granulated by addition of ethyl alcohol to the Collette mixer
4. The wet granulation obtained from step 3 is dried in a fluid bed dryer.
5. The granulation of step 4 is mixed in a Blender.
6. Sieved magnesium stearate is added to blender and mixed with step 5.
7. The lubricated mixture of step 6 is compressed into tablets with a compression machine.

**B. Granules of Outer Tablet**
1. Cetyl Myristate is screened.
2. The step 1 mixture is blended in a blender.
3. The lactose, microcrystalline cellulose, colloidal silicon dioxide, croscarmellose sodium, magnesium stearate and HPMC were screened and added to the blender.
4. The step 3 mixture is blended.
5. Magnesium stearate is screened together with blend of step 4 and blended altogether, then discharged.

**C. Compression as Tablet in Tablet**
- To prepare a tablet-in-tablet composition, granules of outer tablet is compressed onto the internal tablet through Kilian RUD compression machine.

**Table 5**

| **TABLET IN TABLET UNIT FORMULA** | |
|---|---|
| **Ingredients** | **mg** |
| **INTERNAL TABLET** | |
| Cetyl Palmitate | 16.7 |
| Microcrystalline cellulose (AVICEL ® PH 101) | 8 |
| Hydroxypropylmethylcellulose (HPMC K100M Premium CR) | 5 |
| Lactose monohydrate Spray Dried | 7 |

| **OUTER TABLET** | |
|---|---|
| Cetyl Myristate | 333.3 |
| Lactose | 27 |
| Hydroxypropylmethylcellulose (HPMC) | 10 |
| Microcrystalline cellulose | 3 |
| Colloidal silicon dioxide | 7 |
| Croscarmellose sodium | 3 |
| Magnesium Stearate | 4 |
| **Total** | 424 |

### Example 4

**MINI TABLETS IN CAPSULE**

**A.Cetyl Myristate Mini Tablets**
1. Crosscarmellose sodium, cetyl myristate, microcrystalline cellulose are sifted and mixed.
2. Povidone K-30 was dissolved in ethanol and the blend obtained from step1 is granulated using this solution.
3. The granules were dried and sifted.
4. Microcrystalline cellulose, magnesium stearate and Talc were added to the granules and mixed.
5. The blend was then tabletted through Horn-Noack tabletting machine.

**B.Cetyl Palmitate Mini Tablet**
1. Cetyl Palmitate, hydroxypropylcellulose and crospovidone are mixed in a granulator.
2. Mixture is granulated with a hydroxypropylcellulose solution/dispersion and dried in a dryer.
3. The resulting dried granules are mixed with the, crospovidone, corn starch, poloxamer, dicalcium phosphate and mannitol.
4. The resulting blend is mixed with talc and magnesium stearate.
5. The blend was then tabletted through Horn-Noack tabletting machine.

Each cetyl myristate mini tablet contains 33.33 mg cetyl myristate. Various numbers of mini-tablets can be filled into capsules to deliver various capsule strengths. In this invention for 333.3 mg strength cetyl myristate, 10 mini-tablets are placed in one capsule.

One cetyl palmitate mini-tablet and ten cetyl myristate mini-tablets are filled into a hard gelatin capsule.

Mini tablets are filled into capsule via Modu-C (HarroHofliger).

**Table 4**

| **MINI TABLETS IN CAPSULE** | |
|---|---|
| **Ingredients** | **mg** |
| **Cetyl Palmitate Mini Tablet (Per Tablet)** | |
| Cetyl Palmitate | 16.7 |
| Hydroxypropylcellulose | 0.6 |
| Crospovidone | 1 |
| Corn starch | 1.3 |
| Poloxamer | 0.4 |
| Dicalcium phosphate | 1.4 |
| Mannitol | 1.1 |

| **Cetyl Myristate Mini Tablet (Per Tablet)** | |
|---|---|
| Cetyl Myristate | 33.33 |
| Cross Carmellose sodium | 1.3 |
| Microcrystalline cellulose | 1.8 |
| Povidone | 0.9 |
| Magnesium stearate | 1.7 |
| Talc | 2.1 |
| **Total** | 433.8 |

### Example 5

**CAPSULE IN CAPSULE**

Cetyl palmitate granules, prepared pursuant to Example 2, are filled into smaller size capsule. Smaller size capsule is encapsulated into a larger capsule wherein large capsule is also simultaneously filled with cetyl myristate granules. Capsule in capsule operations is performed via Modu-C (HarroHofliger).

## Claims

1. A tablet in tablet of cetyl myristate and cetyl palmitate combination.

2. Tablet-in-tablet composition as claimed in claim one comprises: a) a core tablet comprising: cetyl myristate and an acceptable carrier or carriers and b) a compressed outer tablet layer comprising: cetyl palmitate and an acceptable carrier or carriers or: a) a core tablet comprising cetyl palmitate and an acceptable carrier or carriers and b) a surrounding outer tablet applied thereover comprising cetyl myristate and an acceptable carrier or carriers.

3. An inlay tablet of cetyl myristate and cetyl palmitate combination.

4. Inlay tablet as claimed in claim 3 comprises (a) an inner inlayed tablet comprising cetyl palmitate and optionally acceptable excipient (s); and (b) an outer tablet comprising cetyl myristate and optionally acceptable excipient(s) or: (a) an inner inlayed tablet comprising cetyl myristate optionally acceptable excipient(s); and (b) an outer tablet comprising cetyl palmitate and optionally acceptable excipient(s).

5. A bilayer tablet of cetyl myristate and cetyl palmitate combination.

6. Bilayer tablet as claimed in claim 5 comprises; a) a first layer containing cetyl myristate and optionally acceptable carrier or carriers and b) a second layer containing cetyl palmitate and optionally acceptable carrier or carriers or: a) a first layer containing cetyl palmitate and optionally acceptable carrier or carriers and b) a second layer containing cetyl myristate and optionally acceptable carrier or carriers.

7. A kit of cetyl myristate and cetyl palmitate combination.

8. The kit as claimed in claim 7 comprises: a) therapeutically effective amount cetyl palmitate and optionally an acceptable carrier or diluent in a unit dosage form and b) therapeutically effective amount of cetyl myristate and an acceptable carrier or diluent in a unit dosage form and c) a container.

9. Dosage forms in the kit as claimed in claim 8 are simultaneously or sequentially administered.

10. A capsule in capsule of cetyl myristate and cetyl palmitate combination.

11. Capsule in capsule as claimed in claim 10 wherein smaller size capsule is encapsulated into a larger capsule.

12. Capsule in capsule as claimed in claim 11 wherein smaller size capsule is filled with cetyl palmitate and optionally excipients and larger capsule is filled with cetyl myristate and optionally excipients.

13. A pharmaceutical and/or dietary supplement composition of cetyl myristate and cetyl palmitate **characterized in that** cetyl myristate and cetyl palmitate are dispersed within its own carrier or mixtures of carriers.

14. As claimed in claim 13 each dispersion of cetyl myristate and cetyl palmitate within its carrier or mixtures of carriers is provided following manners: a) Cetyl myristate and cetyl palmitate are separately granulated and then they are separately compressed into mini tablets and filled into capsule altogether or: b) Cetyl myristate and cetyl palmitate are separately granulated and then they are separately compressed into tablet and filled into capsule or: c) One of the active agents, cetyl palmitate or cetyl palmitate, is granulated and compressed into tablet or mini-tablets and then combined with granules of other active agent, cetyl palmitate or cetyl myristate, into capsule or: d) Cetyl myristate and cetyl palmitate are separately granulated and then directly filled into capsule without tabletting or: e) One of the active agents, cetyl palmitate or cetyl palmitate, is prepared through direct compression into tablet or mini-tablet and then combined with granules of other active agent, cetyl palmitate or cetyl myristate, into capsule.

15. A pharmaceutical or dietary supplement composition of cetyl myristate and cetyl palmitate **characterized in that** one of the active agents, cetyl myristate or cetyl palmitate, is granulated and then combined with the other, cetyl myristate or cetyl palmitate, which is not in granulated form.
